# EUROPÄISCHE PATENTANMELDUNG

(11) **EP 4 248 932 A1**
(43) Veröffentlichungstag der Anmeldung: **27.09.2023**
(21) Anmeldenummer: 23163843.8
(22) Anmeldetag: 23.03.2023
(51) Int. Cl.: A61H 1/02

(54) **VORRICHTUNG ZUM AUSÜBEN EINER ZUGKRAFT AUF EIN BEIN**

(30) Priorität: 24.03.2022 AT 501902022
(71) Anmelder: Schusser, Martin, 9523 Landskron (AT)
(72) Erfinder: Schusser, Martin, 9523 Landskron (AT)
(74) Vertreter: Beer & Partner Patentanwälte KG

(57) **Zusammenfassung**

Eine Vorrichtung (1) zum Ausüben einer Zugkraft auf ein Bein (2) umfasst eine oberhalb eines Fußes (4) anordenbare Manschette (3) sowie ein Zugelement (5). Das Zugelement (5) ist an einem ersten Ende (6) mit der Manschette (3) verbunden und an einem zweiten Ende (7) ortsfest fixierbar. Das Zugelement (5) ist ausgehend von einem Ausgangszustand in seine Längsrichtung (L) elastisch verlängerbar. Das Zugelement (5) weist einen Außenkörper (8), der mit einem der Enden (6, 7) verbunden ist, und wenigstens einen mit dem anderen der Enden (7, 6) verbundenen Innenkörper (9) auf, der beim Längenverändern des Zugelementes (5) zumindest bereichsweise im Außenkörper (8) verschiebbar ist. Der Innenkörper (8) weist einen Anzeigebereich (14) und der Außenkörper (9) eine Markierung (16) oder der Außenkörper (8) weist einen Anzeigebereich (14) und der Innenkörper (9) eine Markierung (16) auf. Die Markierung (16) ist im Ausgangszustand außerhalb des Anzeigebereiches (14) angeordnet und ist in einem Verwendungszustand, bei dem das Zugelement (5) aufgrund einer darauf wirkenden Zugkraft um eine vorbestimmte Länge (ΔL) gegenüber dem Ausgangszustand verlängert ist, an oder in dem Anzeigebereich (14) angeordnet.

## Beschreibung

Die Erfindung betrifft eine Vorrichtung zum Ausüben einer Zugkraft auf ein Bein, die eine oberhalb eines Fußes anordenbare Manschette sowie ein Zugelement umfasst, wobei das Zugelement an einem ersten Ende mit der Manschette verbunden und an einem zweiten Ende ortsfest fixierbar ist, wobei das Zugelement ausgehend von einem Ausgangszustand in seine Längsrichtung elastisch verlängerbar ist und wobei das Zugelement einen Außenkörper, der mit einem der Enden verbunden ist, und wenigstens einen mit dem anderen der Enden verbundenen Innenkörper aufweist, der beim Längenverändern des Zugelementes zumindest bereichsweise im Außenkörper verschiebbar ist.

Derartige Vorrichtungen zum Ausüben einer Zugkraft auf ein Bein kommen oft nach einer Operation am Bein oder bei einer chronischen Erkrankung des Sprung-, Knie- oder Hüftgelenks, wie einer Arthrose, zum Einsatz. Durch das Ausüben der Zugkraft auf das Bein werden Sehnen und Bänder, die Gelenke stabilisieren und die Gelenksknorpel aufeinanderpressen, gedehnt. Das Dehnen der Bänder und Sehnen vermindert den von den Gelenksflächen aufeinander ausgeübten Druck, sodass die Gelenke zumindest zeitweise entlastet werden.

Besonders vorteilhaft ist es, wenn Personen, die in regelmäßigen Abständen eine Entlastung der Beingelenke oder eines Beingelenkes benötigen - beispielsweise da sie an einer Arthrose des Kniegelenkes leiden - diese Entlastung auf möglichst einfache Weise selber herbeiführen können.

Für diesen Zweck gibt es bereits unterschiedliche Vorrichtungen, die es einer Person erlauben, ohne Zutun von anderen Personen eine Zugkraft auf das zu entlastende Bein auszuüben.

Diese Vorrichtungen weisen in der Regel eine Bein- bzw. Fußmanschette auf, die um das zu entlastende Bein gelegt wird und die mit einem Ende eines Zugelementes, wie einem Seil, verbunden ist. Indem entweder die Person ortsfest verharrt und das andere Ende des Zugelementes von der Person wegbewegt wird (z.B. mittels eines von der Person bedienbaren Seilzuges) oder indem das andere Ende des Zugelementes ortsfest fixiert wird und sich die Person von diesem wegbewegt, wird eine Zugkraft, die zur Entlastung der Gelenke bzw. des Gelenkes notwendig ist, auf das Bein ausgeübt.

Aus US 2014/315695 A1, US 2009/299248 A1 und DE 103 34 668 A1 sind Vorrichtungen zum Ausüben einer Zugkraft auf ein Bein für therapeutische Zwecke bekannt.

Nachteilig an bekannten Vorrichtungen ist deren komplizierter Aufbau, sodass sich diese Vorrichtungen kaum dafür eignen, auf Reisen mitgenommen zu werden.

Ein weiterer großer Nachteil bekannter Vorrichtungen liegt darin, dass die die Vorrichtung nutzende Person nur sehr schwer einzuschätzen kann, zu welchem Zeitpunkt bzw. in welchem Verwendungszustand der Vorrichtung eine geeignete Zugkraft auf das Bein ausgeübt wird. Das richtige Abschätzen der Zugkraft ist jedoch besonders wichtig, da bei einer zu geringen Zugkraft keine ausreichende Entlastung der Gelenke auftritt. Ebenso besteht die Gefahr, dass unbewusst eine zu hohe Zugkraft auf das Bein ausgeübt wird, was gravierende Folgen für die Sehnen und Bänder der ohnedies schon beanspruchten Gelenke haben kann.

Der Erfindung liegt daher die Aufgabe zu Grunde, eine Vorrichtung der eingangs genannten Gattung zur Verfügung zu stellen, die die Nachteile des Standes der Technik nicht aufweist. Insbesondere soll eine Vorrichtung bereitgestellt werden, mit der eine Person möglichst einfach und effektiv und ohne fremde Hilfe eine geeignete Zugkraft auf ihr eigenes Bein ausüben kann, um beispielsweise das Kniegelenk zu entlasten.

Gelöst wird diese Aufgabe erfindungsgemäß mit einer Vorrichtung, die die Merkmale von Anspruch 1 aufweist.

Bevorzugte und vorteilhafte Ausführungsformen der Erfindung sind Gegenstand der Unteransprüche.

Erfindungsgemäß ist vorgesehen, dass der Innenkörper einen Anzeigebereich und der Außenkörper eine Markierung oder der Außenkörper einen Anzeigebereich und der Innenkörper eine Markierung aufweist, und dass die Markierung im Ausgangszustand außerhalb des Anzeigebereiches angeordnet ist und in einem Verwendungszustand, bei dem das Zugelement aufgrund einer darauf wirkenden Zugkraft um eine vorbestimmte Länge gegenüber dem Ausgangszustand verlängert ist, an oder in dem Anzeigebereich angeordnet ist.

Durch das Anlegen der Manschette und das Fixieren des zweiten Endes des Zugelementes an einem ortsfesten Punkt, z.B. an einer Tür oder einem schweren Möbelstück, kann die die Manschette tragende Person eine Zugkraft auf das Zugelement ausüben und das Zugelement dehnen, indem sie sich vom ortsfesten Punkt wegbewegt. Da das Zugelement elastisch verlängerbar ist, baut sich durch das Dehnen des Zugelementes eine Federkraft auf, die gegengleich groß zur auf das Zugelement ausgeübten Zugkraft ist. Die Federkraft, die auch als Zugkraft bezeichnet werden kann, wirkt auf das die Manschette tragende Bein der Person und zieht dieses in die Länge. Durch die Federkraft werden daher die Bänder und Sehnen des Beins gedehnt, wodurch eine Entlastung der Gelenke, insbesondere des Kniegelenkes, stattfindet.

Die erfindungsgemäße Vorrichtung ermöglicht Personen, besonders schnell und einfach und ohne fremde Hilfe für eine zeitweilige Entlastung der Gelenke im Bein zu sorgen. Der unkomplizierte und robuste Aufbau der erfindungsgemäßen Vorrichtung, die bevorzugt lediglich mechanische Komponenten aufweist, ermöglicht Personen, die Vorrichtung überall hin mitzunehmen (z.B. auch auf Reisen oder zum Wandern), ohne Angst haben zu müssen, dass die Vorrichtung beschädigt wird oder vor Ort nicht einsetzbar ist.

Als Anzeigebereich wird im Rahmen der Erfindung ein markierter (d.h. hervorgehobener) Bereich angesehen, der von einem oder mehreren daran angrenzenden Bereich/en abgegrenzt ist. Die Abgrenzung kann beispielsweise durch eine besondere optische Hervorhebung, insbesondere durch Anbringen von Zeichen im Bereich oder Einfärben des Bereiches, eine besondere Oberflächengestaltung oder durch Abgrenzungslinien bzw. Abgrenzungszeichen an den Bereichsgrenzen erfolgen.

Die Markierung kann eine markante, konstruktionsbedingte Stelle an der Vorrichtung, wie beispielsweise eine Kante, ein Fortsatz oder eine Ausnehmung sein, kann aber auch eine mittels Zeichen farblich oder anders hervorgehobene Stelle an der Vorrichtung sein. Weiters kann die Markierung im Wesentlichen punktförmig oder strichförmig, aber auch bereichsförmig sein.

Im Ausgangszustand, d.h. in einem unbelasteten Zustand der Vorrichtung, in dem das Zugelement nicht gedehnt ist, ist die Markierung außerhalb des Anzeigebereiches angeordnet. Durch Dehnung, d.h. Längenveränderung, des Zugelementes nähert sich die Markierung dem Anzeigebereich an und tritt in den Anzeigebereich ein, sobald die Längenveränderung des Zugelementes einen unteren Grenzwert erreicht. Die Positionierung des Anzeigebereiches bzw. die Festlegung des unteren Grenzwertes erfolgt in Kenntnis der Dehnungseigenschaften des Zugelementes und hängt davon ab, bei welcher Längenveränderung des Zugelementes die gewünschte, vom Zugelement auszuübende, Federkraft (bzw. Zugkraft) erreicht ist. Die Stärke der gewünschten Federkraft hängt vom Anwendungsfall ab und entspricht jener Kraft, die notwendig ist, um das Bein der die Vorrichtung nutzenden Person so stark zu dehnen, dass die Gelenke bzw. zumindest ein bestimmtes Gelenk entlastet werden/wird.

Die Vorrichtung kann speziell für bestimmte Personengruppen (Männer, Frauen, Kinder, Personen über oder unter 175 cm Körpergröße, Personen mit Kniegelenksschäden, Personen mit Hüftgelenksschäden, etc.) konzipiert sein oder sogar extra für bestimmte Einzelpersonen angefertigt werden. Wichtig dabei ist, dass die Federkraft (bzw. Zugkraft), die von der Zugvorrichtung auf das Bein ausgeübt wird, wenn die Markierung im Anzeigebereich angeordnet ist, den Bedürfnissen und körperlichen Voraussetzungen der Personengruppe bzw. Person entspricht.

Insbesondere ist im Rahmen der Erfindung eine Ausführungsform bevorzugt, bei der das Zugelement ein elastisch, insbesondere hochelastisch, verformbares Dehnelement aufweist, das am oder im Innenkörper angeordnet oder der Innenkörper selbst ist. Wenn das Dehnelement der Innenkörper selbst ist, kann der Anzeigebereich bzw. die Markierung direkt auf dem Dehnelement angeordnet sein.

Vorzugsweise ist das Dehnelement der einzige elastisch bzw. hochelastisch verformbare Bestandteil des Zugelementes, wobei der Außenkörper im Wesentlichen unelastisch bzw. starr ist. Bei Ausführungsformen, in denen der Innenkörper ein vom Dehnelement separater Bauteil ist, ist es ebenfalls bevorzugt, wenn der Innenkörper im Wesentlichen unelastisch bzw. starr ist.

In einer bevorzugten Weiterbildungsform ist vorgesehen, dass der Außenkörper rohrförmig ist, dass der Innenkörper rohrförmig und zumindest abschnittsweise im Außenkörper aufgenommen ist, und dass das Dehnelement zumindest abschnittsweise im Innenkörper und im Außenkörper, vorzugsweise vom einen zum anderen Ende des Zugelementes verlaufend, angeordnet ist. Bei derartigen Ausführungsformen ist das Dehnelement ein vom Innenkörper separater Bauteil. Da der Außenkörper mit einem Ende des Zugelementes und der Innenkörper mit dem anderen Ende des Zugelementes fix verbunden ist und das Dehnelement, das bei derartigen Ausführungsformen vorzugsweise mit beiden Enden verbunden ist, elastisch verformbar ist, verschieben sich der Außenkörper und der Innenkörper zueinander bzw. gegeneinander, wenn das Dehnelement gedehnt wird.

Denkbar ist im Rahmen der Erfindung auch, dass das Dehnelement nicht mit beiden Enden des Zugelementes verbunden ist, sondern nur mit einem Ende und dem Innenkörper (oder den Außenkörper), oder nur mit dem Innenkörper und dem Außenkörper, aber nicht mit einem der Enden des Zugelementes.

Im Rahmen der Erfindung ist das Dehnelement vorzugsweise ein elastisch dehnbarer Bauteil, der derart ausgestaltet und im Zugelement angeordnet ist, dass er bei einer Längenveränderung des Zugelementes gedehnt wird. Das Dehnelement kann jedoch auch derart ausgestaltet und angeordnet sein, dass es im Zuge der Längenveränderung des Zugelementes elastisch gestaucht wird.

Bevorzugt besteht das Dehnelement zumindest teilweise aus einem Kunststoff, vorzugsweise einem Elastomer, insbesondere aus Gummi. Beispielsweise kann der Kunststoff bzw. das Elastomer, aus dem das Dehnelement besteht, einen E-Modul von < 5 GPa, vorzugsweise < 1 GPa, insbesondere <0,1 GPa aufweisen. Wenn es aus Gummi besteht, hat es beispielsweise einen E-Modul von ca. 0,05 GPa. Derartige Materialeigenschaften werden im Rahmen der Erfindung als elastisch bzw. hochelastisch verformbar angesehen.

Das Dehnelement kann gänzlich oder zumindest teilweise aus Kunststoff bestehen. Im Rahmen der Erfindung ist es jedoch auch möglich, dass das Dehnelement eine Feder, insbesondere aus Metall, ist.

Bevorzugt sind Ausführungsformen, bei denen die vorbestimmte Länge, um die das Zugelement gegenüber dem Ausgangszustand verlängert ist, wenn die Markierung im Verwendungszustand im oder am Anzeigebereich angeordnet ist, größer oder gleich einem unteren Grenzwert ist. In der Praxis ist es nicht unbedingt notwendig, dass die gewünschte Federkraft bzw. Zugkraft, die die Vorrichtung auf das Bein ausüben soll, ganz exakt einen bestimmten Betrag aufweisen muss. Vielmehr reicht es in der Regel aus, wenn der Betrag der Zugkraft einen Mindestwert übersteigt, und - während die Vorrichtung verwendet wird - in einem bestimmten Wertebereich liegt. Wichtig ist vor allem, dass die Zugkraft einen unteren Kraftgrenzwert erreicht, da unter diesem Kraftwert die Gelenke nicht entlastet werden. Vorzugsweise ist jedoch auch vorgesehen, dass die vorbestimmte Länge kleiner oder gleich einem oberen Grenzwert ist, damit die Zugkraft - solange die vorbestimmte Länge in dem vorgesehenen Anzeigebereich liegt - keinen für die Gelenke schädlichen Kraftwert erreicht.

Im Rahmen der Erfindung ist es möglich, dass der Innenkörper bzw. der Außenkörper wenigstens einen weiteren Anzeigebereich aufweist, und dass die Markierung an oder in dem weiteren Anzeigebereich bzw. ggf. in weiteren Anzeigebereichen angeordnet ist, wenn das Zugelement gegenüber dem Ausgangszustand um eine weitere (bzw. andere) vorbestimmte Länge bzw. ggf. um jeweils eine weitere vorbestimmte Länge verlängert ist. Die weitere (bzw. andere) vorbestimmte Länge, um die das Zugelement verlängert sein muss, damit die Markierung im weiteren Anzeigebereich angeordnet ist, ist länger oder kürzer als die vorbestimmte Länge, um die das Zugelement verlängert sein muss, damit die Markierung im (ursprünglichen) Anzeigebereich angeordnet ist.

Im Rahmen der Erfindung können auch mehr als zwei, beispielsweise drei, vier oder mehr als vier, Anzeigebereiche vorgesehen sein. Möglich ist auch, dass der (ursprüngliche) Anzeigebereich innerhalb eines weiteren Anzeigebereichs angeordnet bzw. "eingebettet" ist.

Der weitere bzw. die weiteren Anzeigebereich/e ermöglicht/ermöglichen zum einen, dass die Vorrichtung für unterschiedliche Übungen oder Personengruppen eingesetzt werden kann, und zum anderen, dass die Vorrichtung unmissverständlich anzeigt, ob eine unerwünschte Zugkraft ausgeübt wird.

Im Rahmen der Erfindung ist es ebenfalls möglich, dass am Innenkörper oder am Außenkörper eine Skala angeordnet, insbesondere aufgedruckt oder aufgeklebt, ist. Die Striche oder Punkte der Skala begrenzen dabei eine Vielzahl an Anzeigebereichen, wobei jeweils zwischen zwei benachbarten Strichen bzw. Punkten der Skala ein separater Anzeigebereich gebildet ist.

Auch die weitere vorbestimmte Länge, um die das Zugelement gegenüber dem Ausgangszustand verlängert ist, wenn die Markierung im oder am weiteren Anzeigebereich angeordnet ist, kann im Rahmen der Erfindung größer oder gleich einem weiteren unteren Grenzwert und vorzugsweise kleiner oder gleich einem weiteren oberen Grenzwert sein.

Bevorzugt sind Ausführungsformen, bei denen der Innenkörper den bzw. ggf. die Anzeigebereich/e aufweist, und der bzw. ggf. die Anzeigebereich/e im Ausgangszustand vom Außenkörper verdeckt und insbesondere nicht sichtbar ist/sind. Bei derartigen Ausführungsformen gleitet der Innenkörper (der auch das Dehnelement sein kann oder aber ein vom Dehnelement separates Bauteil ist) beim Längenverändern des Zugelementes an einer Seite aus dem Außenkörper hinaus. Durch das Hinausgleiten werden zuerst vom Außenkörper verdeckte Bereiche mit dem/den Anzeigebereich/en sichtbar. Denkbar ist jedoch auch, dass der Außenkörper eine Öffnung, beispielsweise ein Fenster, als Markierung aufweist, und dass der Anzeigebereich, der davor vom Außenkörper verdeckt war, durch das Gleiten des Innenkörpers im Außenkörper beim Erreichen der vorbestimmten Länge in der Öffnung sichtbar wird.

In einer bevorzugten Ausführungsform ist die Markierung an jenem Ende des Außenkörpers, das in Richtung des mit dem Innenkörper verbundenen Endes des Zugelementes ragt, angeordnet. Insbesondere kann die Markierung der Abschlussrand dieses Endes sein.

Besonders bevorzugt ist eine Ausführungsform, bei der die Markierung durch den Außenrand des Außenkörpers gebildet ist und bei der der/die Anzeigebereich/e am Innenkörper angeordnet ist/sind und im Ausgangszustand vom Außenkörper verdeckt ist/sind. Durch Längenverändern des Zugelementes tritt ab einer bestimmten Zugkraft der Bereich des Innenkörpers mit dem Anzeigebereich aus dem Außenkörper hervor und überragt den Außenrand des Außenkörpers.

Im Rahmen der Erfindung ist insbesondere vorgesehen, dass der Anzeigebereich optisch hervorgehoben ist. Insbesondere kann er in einer Signalfarbe eingefärbt sein. Wenn mehrere Anzeigebereiche vorhanden sind, kann/können einer, mehrere oder alle Anzeigebereiche eingefärbt sein. Beispielsweise können unterschiedliche Anzeigebereiche jeweils in einer anderen Signalfarbe eingefärbt sein.

Denkbar ist z.B., dass ein Anzeigebereich, der grün eingefärbt ist, in einem weiteren Anzeigebereich, der rot eingefärbt ist, eingebettet ist. Beim Längenverändern des Zugelementes ist die Markierung zuerst im roten Anzeigebereich angeordnet. Erst wenn die Längenveränderung einen unteren Grenzwert erreicht, ist die Markierung im grünen Bereich angeordnet. Beim Überschreiten eines oberen Grenzwertes für die Längenveränderung ist die Markierung wieder im roten Bereich angeordnet. Die Färbung zeigt einer die Vorrichtung benutzenden Person schnell und unmissverständlich an, ob die Längenveränderung des Zugelementes und damit der Betrag der auf das Bein ausgeübten Federkraft bzw. Zugkraft in einem passenden Wertebereich liegt, oder ob eine zu geringe oder zu hohe Zugkraft auf das Bein ausgeübt wird.

Im Rahmen der Erfindung kann vorgesehen sein, dass die Manschette einen bandförmigen Gurtbereich, mit dem das Bein oberhalb des Fußes zumindest teilweise umschließbar ist, und einen damit verbundenen bandförmigen Rückenbereich, der hinten an der Ferse des Fußes anordenbar ist, aufweist, und dass der Rückenbereich an einem vom Gurtbereich beabstandeten Ende mit dem Zugelement verbunden ist.

Vorzugsweise ist der geschlossene Gurtbereich in seinem Umfang veränderbar, d.h. größenverstellbar, sodass ein variabel großer Bereich vom Gurtbereich eng umschließbar ist. Insbesondere ist der Gurtbereich durch einen Gurt gebildet, der mit Hilfe eines Verschlusses, wie einer Schnalle oder einem Klettverschluss, enger oder weiter gestellt werden kann. Die Vorrichtung kann somit von anatomisch unterschiedlich geformten Personen getragen werden. Möglich ist bei derartigen Ausführungsformen auch, dass die Manschette am Bein nicht direkt oberhalb des Fußes, sondern direkt oberhalb des Knies getragen wird.

Besonders bevorzugt ist es, wenn der Rückenbereich der Manschette eine Materialaussparung bzw. eine Aussparung mit verringerter Materialstärke aufweist, in welche die Ferse des Fußes bei einer am Bein angelegten und dieses zumindest teilweise umschließenden Manschette platzierbar ist. Die Federkraft bzw. Zugkraft, die vom Zugelement auf die Manschette und damit von der Vorrichtung auf das Bein ausgeübt wird, wird bei einer derartigen Ausführungsform nicht über die Ferse geleitet, was aus medizinischer Sicht und hinsichtlich des Tragekomforts besonders vorteilhaft ist.

Die Manschette kann im Rahmen der Erfindung aus Leder, Stoff oder Kunststoff, wie Kunstleder oder Neopren, aber auch aus anderen passenden Materialien geformt sein.

Weitere Einzelheiten, Merkmale und Vorteile der Erfindung ergeben sich aus der nachstehenden Beschreibung unter Bezugnahme auf die angeschlossenen Zeichnungen, in welchen eine bevorzugte Ausführungsform dargestellt ist. Es zeigt:
- Fig. 1: eine isometrische Ansicht der erfindungsgemäßen Vorrichtung in einem Zwischenzustand,
- Fig. 2: eine isometrische Ansicht der erfindungsgemäßen Vorrichtung in einem Verwendungszustand, und
- Fig. 3: eine Draufsicht auf die geöffnete, nicht an einem Bein angeordnete, Manschette und das Zugelement der Vorrichtung in einem Ausgangszustand.

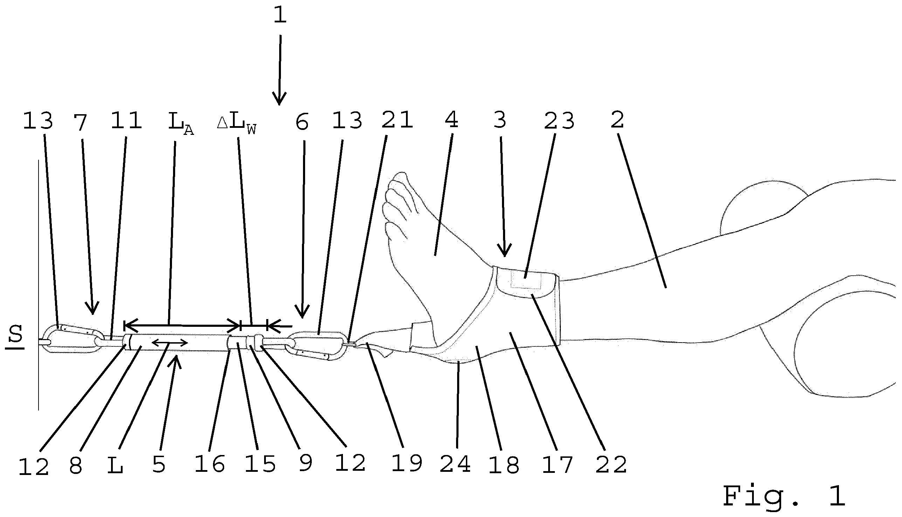

Die Fig. 1, 2 und 3 zeigen eine erfindungsgemäße Vorrichtung 1 gemäß einer bevorzugten Ausführungsform.

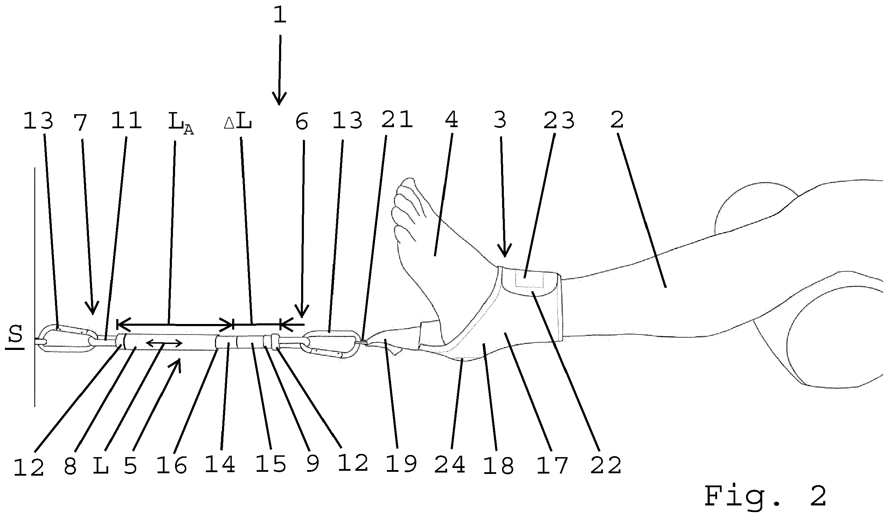

In den Fig. 1 und 2 ist die Vorrichtung 1 in einer isometrischen Ansicht und an einem Bein 2 einer Person angeordnet bzw. mit diesem verbunden dargestellt.

Die Vorrichtung 1 weist eine Manschette 3 auf, die oberhalb des Fußes 4 am Bein 2 einer die Vorrichtung nutzenden Person angeordnet ist. Der Fuß 4 verhindert, dass die Manschette 3 vom Bein 2 rutscht, während von der Vorrichtung 1 eine Zugkraft bzw. Federkraft auf das Bein 2 ausgeübt wird.

Weiters weist die Vorrichtung 1 ein Zugelement 5 auf, das an einem ersten Ende 6 mit der Manschette 3 verbunden ist. An einem zweiten Ende 7 ist das Zugelement 5 an einer ortsfesten Stelle S fixiert, beispielsweise an einer Türe oder einem schweren Möbelstück.

In Fig. 1 ist die Vorrichtung 1 bzw. das Zugelement 5 in einem Zwischenzustand und in Fig. 2 in einem Verwendungszustand dargestellt.

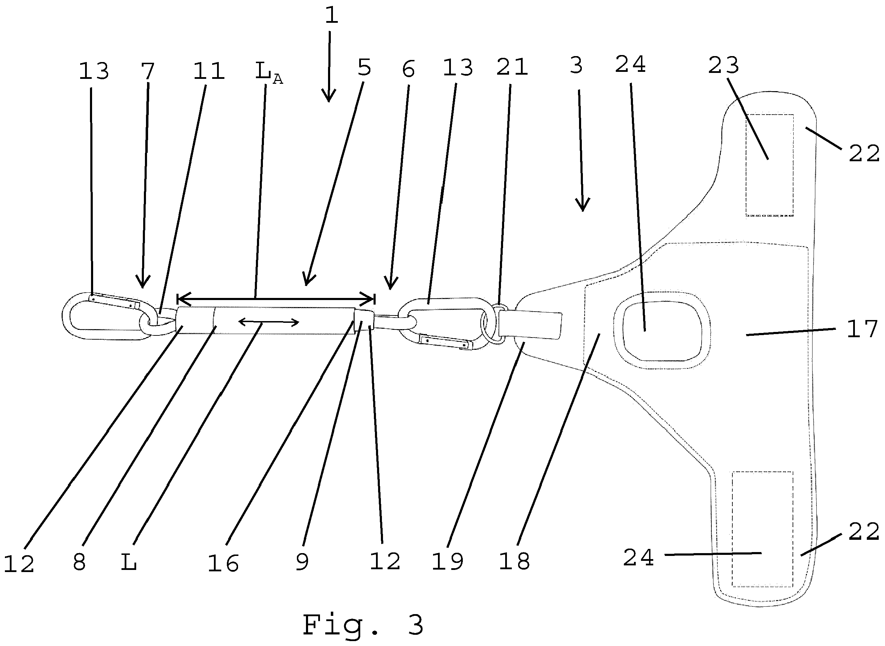

In Fig. 3 ist die Vorrichtung 1 in einem nicht am Bein 2 und der ortsfesten Stelle S angeordneten Zustand, in dem die Manschette 3 unverschlossen ist, dargestellt. Das Zugelement 5 ist in Fig. 3 unverlängert dargestellt, d.h. in einem Ausgangszustand bzw. mit jener Anfangslänge L_{A}, die es im dargestellten Ausgangszustand der Vorrichtung 1 aufweist.

Das Zugelement 5 weist einen rohrförmigen Außenkörper 8 auf, in dem zumindest abschnittsweise ein Innenkörper 9 angeordnet ist.

Durch den Innenkörper 9 - und auch durch den Außenkörper 8 - ist ein Dehnelement 11 geführt, das in der dargestellten Ausführungsform ein elastisches Gummiband ist.

Die Enden des Dehnelementes 11 sind in der dargestellten Ausführungsform gleichzeitig die Enden 6, 7 des Zugelementes 5.

In der dargestellten Ausführungsform ist der Außenkörper 8 mit dem zweiten Ende 7 und der Innenkörper 9 mit dem ersten Ende 6 - im Wesentlichen starr - verbunden. Die Verbindung 12 zwischen Außenkörper 8 und zweitem Ende 7 (d.h. dem einen Ende des Dehnelementes 11) ist beispielsweise eine Schrumpf- oder Klebeverbindung, genauso wie die Verbindung 12 zwischen dem Innenkörper 9 und dem ersten Ende 6 (d.h. dem anderen Ende des Dehnelementes 11).

Sowohl das erste als auch das zweite Ende 6, 7 ist über Verbindungsmittel 13, wie Karabiner, mit der Manschette 3 bzw. der ortsfesten Stelle S verbunden bzw. verbindbar.

In der dargestellten Ausführungsform weist der Innenkörper 9 einen Anzeigebereich 14 und einen weiterer Anzeigebereich 15 auf. Die Anzeigebereiche 14, 15 sind optisch hervorgehoben, beispielsweise indem eine Oberfläche des Innenkörpers 9 im Anzeigebereich 14 grün und im weiteren Anzeigebereich 15 rot eingefärbt ist.

Durch ein Wegbewegen des Beins 2 von der ortsfesten Stelle S wird das elastische Dehnelement 11 gedehnt, sodass das Zugelement 5 in seine Längsrichtung L elastisch verlängert wird.

In Fig. 3 ist das Zugelement 5 in seinem Ausgangszustand und daher in seine Längsrichtung L unverlängert dargestellt (d.h. wie im Ausgangszustand der Vorrichtung). Dabei weist es seine Ausgangslänge L_{A} auf. Die Anzeigebereiche 14, 15 sind in diesem unverlängerten Zustand vom Außenkörper 8 verdeckt.

In Fig. 1 ist das Zugelement 5 bzw. ist die Vorrichtung 1 in einem Zwischenzustand dargestellt, in dem das Zugelement 5 gegenüber seinem Ausgangszustand und seiner Ausgangslänge L_{A} verlängert ist.

Der zum ersten Ende 6 des Zugelementes 5 weisende Abschlussrand des rohrförmigen Außenkörpers 8 dient als Markierung 16, wobei diese Markierung 16 im in Fig. 1 dargestellten Zwischenzustand - in dem das Zugelement 5 gegenüber seinem Ausgangszustand bzw. seiner Ausgangslänge L_{A} um eine weitere vorbestimmte Länge ΔL_{W} verlängert ist - im rot eingefärbten, weiteren Anzeigebereich 15 angeordnet ist.

In Fig. 2 ist das Zugelement 5 so weit in seine Längsrichtung L verlängert, d.h. um die vorbestimmte Länge ΔL, dass die Markierung 16 im grün eingefärbten Anzeigebereich 14 angeordnet ist. Die vorbestimmte Länge ΔL, um die das Zugelement 5 gegenüber dem Ausgangszustand (d.h. auch gegenüber der Ausgangslänge L_{A}) verlängert ist, ist dabei gleich groß oder größer einem unteren Grenzwert.

Die Manschette 3 weist einen bandförmigen Gurtbereich 17 auf, mit dem das Bein 2 oberhalb des Fußes 4 wie mit einem Gurt umschließbar ist.

Der Gurtbereich 17 ist mit einem bandförmigen Rückenbereich 18 verbunden, der im Wesentlichen quer vom Gurtbereich 17 weg verläuft. An einem vom Gurtbereich 17 beabstandeten Ende 19 weist der Rückenbereich 18 ein Anschlussmittel 21, wie einen Ring, auf. An diesem Anschlussmittel ist die Manschette 3 mit dem Zugelement 5, z.B. über das als Karabiner ausgeführte Verbindungsmittel 13, verbunden. Bei der an das Bein 2 angeordneten Vorrichtung 1 verläuft der Rückenbereich 18 hinten an der Ferse des Fußes 4 entlang und in Längsrichtung L des Zugelementes 5.

Der Gurtbereich 17 weist zwei Gurtenden 22 auf, die zum Umschließen des Beins 2 über einen Verschluss 23 miteinander verbunden werden. Der Verschluss 23 ist beispielsweise wie dargestellt ein Klettverschluss, sodass der Gurtbereich 17 enger oder weiter gestellt werden kann, d.h. einen variabel großen Bereich eng umschließen kann.

Der Rückenbereich 18 weist in jenem Bereich, in dem bei der angelegten Manschette 3 die Ferse des Fußes 4 angeordnet ist, eine Aussparung 24 auf. Diese Aussparung 24 ist entweder ein Bereich mit verringerter Materialstärke, oder aber ein Durchgangsloch, sodass in diesem Bereich gar kein Material vorhanden ist.

Im Rahmen der Erfindung können auch andere Verbindungsmittel 13 als Karabiner sowie Anschlussmittel 21 verwendet werden, um das Zugelement 5 mit der Manschette 3 und/oder das Zugelement 5 mit der ortsfesten Stelle S zu verbinden. Beispielsweise kann das Zugelement 5 an einem Ende 6, 7 fest mit der Manschette 3 verbunden, z.B. vernäht oder verklebt, sein. Das Zugelement 5 kann an die ortsfeste Stelle auch angebunden oder angeklemmt sein.

Im Rahmen der Erfindung kann auch vorgesehen sein, dass an den Anzeigebereich 14 bzw. an den weiteren Anzeigebereich 15 ein Bereich angrenzt, der optisch nicht hervorgehoben ist. Beispielsweise ist bei derartigen Ausführungsformen die Markierung 16 bei einer unterhalb eines unteren Grenzwertes liegenden Längenveränderung des Zugelementes 5 in diesem nicht optisch hervorgehobenen - z.B. in weißer Farbe gehaltenen - Bereich angeordnet. Bei der in den Figuren dargestellten Ausführungsform könnte beispielsweise zwischen dem ersten Ende 6 und dem weiteren Anzeigebereich 15 ein optisch nicht hervorgehobener, beispielsweise weißer, Bereich angeordnet sein. Denkbar ist auch, dass der weitere Anzeigebereich 15 durch einen optisch nicht hervorgehobenen Bereich ersetzt ist, sodass die Markierung 16 bei einer kleinen Längenveränderung des Zugelementes 5 im optisch nicht hervorgehobenen Bereich angeordnet ist und erst, wenn das Zugelement 5 von der Ausgangslänge L_{A} um die vorbestimmte Länge ΔL verlängert ist, im Anzeigebereich 14 angeordnet ist.

### Bezugszeichenliste

- 1: Vorrichtung
- 2: Bein
- 3: Manschette
- 4: Fuß
- 5: Zugelement
- 6: erstes Ende (Zugelement)
- 7: zweites Ende (Zugelement)
- 8: Außenkörper
- 9: Innenkörper
- 10: ---
- 11: Dehnelement
- 12: Verbindung
- 13: Verbindungsmittel
- 14: Anzeigebereich
- 15: weiterer Anzeigebereich
- 16: Markierung
- 17: Gurtbereich
- 18: Rückenbereich
- 19: Ende (Rückenbereich)
- 20: ---
- 21: Anschlussmittel
- 22: Gurtende
- 23: Verschluss
- 24: Aussparung

- S: ortsfeste Stelle
- L: Längsrichtung Zugelement
- L_{A}: Ausgangslänge Zugelement
- ΔL: vorbestimmte Länge
- ΔL_{W}: weitere vorbestimmte Länge

## Patentansprüche

1. Vorrichtung (1) zum Ausüben einer Zugkraft auf ein Bein (2), die eine oberhalb eines Fußes (4) anordenbare Manschette (3) sowie ein Zugelement (5) umfasst, wobei das Zugelement (5) an einem ersten Ende (6) mit der Manschette (3) verbunden und an einem zweiten Ende (7) ortsfest fixierbar ist, wobei das Zugelement (5) ausgehend von einem Ausgangszustand in seine Längsrichtung (L) elastisch verlängerbar ist und wobei das Zugelement (5) einen Außenkörper (8), der mit einem der Enden (6, 7) verbunden ist, und wenigstens einen mit dem anderen der Enden (7, 6) verbundenen Innenkörper (9) aufweist, der beim Längenverändern des Zugelementes (5) zumindest bereichsweise im Außenkörper (8) verschiebbar ist, **dadurch gekennzeichnet, dass** der Innenkörper (9) einen Anzeigebereich (14) und der Außenkörper (8) eine Markierung (16) oder der Außenkörper (8) einen Anzeigebereich (14) und der Innenkörper (9) eine Markierung (16) aufweist, und dass die Markierung (16) im Ausgangszustand außerhalb des Anzeigebereiches (14) angeordnet ist und in einem Verwendungszustand, bei dem das Zugelement (5) aufgrund einer darauf wirkenden Zugkraft um eine vorbestimmte Länge (ΔL) gegenüber dem Ausgangszustand verlängert ist, an oder in dem Anzeigebereich (14) angeordnet ist.

2. Vorrichtung nach Anspruch 1, **dadurch gekennzeichnet, dass** das Zugelement (5) ein elastisch, insbesondere hochelastisch, verformbares Dehnelement (11) aufweist, das am oder im Innenkörper (9) angeordnet, oder der Innenkörper (9) selbst ist.

3. Vorrichtung nach Anspruch 2, **dadurch gekennzeichnet, dass** der Außenkörper (8) rohrförmig ist, dass der Innenkörper (9) rohrförmig und zumindest abschnittsweise im Außenkörper (8) aufgenommen ist, und dass das Dehnelement (11) zumindest abschnittsweise im Innenkörper (9) und im Außenkörper (8), vorzugsweise vom einen zum anderen Ende (6, 7) des Zugelementes (5) verlaufend, angeordnet ist.

4. Vorrichtung nach Anspruch 2 oder 3, **dadurch gekennzeichnet, dass** das Dehnelement (11) zumindest teilweise aus einem Elastomer, insbesondere aus Gummi, besteht.

5. Vorrichtung nach einem der Ansprüche 1 bis 4, **dadurch gekennzeichnet, dass** die vorbestimmte Länge (ΔL), um die das Zugelement (5) gegenüber dem Ausgangszustand verlängert ist, wenn die Markierung (16) im Verwendungszustand im oder am Anzeigebereich (14) angeordnet ist, größer oder gleich einem unteren Grenzwert ist und vorzugsweise kleiner oder gleich einem oberen Grenzwert ist.

6. Vorrichtung nach einem der Ansprüche 1 bis 5, **dadurch gekennzeichnet, dass** der Innenkörper (9) bzw. der Außenkörper (8) wenigstens einen weiteren Anzeigebereich (15) aufweist, und dass die Markierung (16) an oder in dem weiteren Anzeigebereich (15) bzw. ggf. in weiteren Anzeigebereichen (15) angeordnet ist, wenn das Zugelement (5) gegenüber dem Ausgangszustand um eine weitere vorbestimmte Länge (ΔL_{W}) bzw. ggf. um jeweils eine weitere vorbestimmte Länge (ΔL_{W}), die gegenüber der vorbestimmten Länge (ΔL) kürzer oder länger ist/sind, verlängert ist.

7. Vorrichtung nach Anspruch 6, **dadurch gekennzeichnet, dass** die weitere vorbestimmte Länge (ΔL_{W}), um die das Zugelement (5) gegenüber dem Ausgangszustand verlängert ist, wenn die Markierung (16) im oder am weiteren Anzeigebereich (15) angeordnet ist, größer oder gleich einem weiteren unteren Grenzwert ist und vorzugsweise kleiner oder gleich einem weiteren oberen Grenzwert ist.

8. Vorrichtung nach einem der Ansprüche 1 bis 7, **dadurch gekennzeichnet, dass** der Innenkörper (9) den bzw. ggf. die Anzeigebereich/e (14, 15) aufweist, und dass der bzw. ggf. die Anzeigebereich/e (14, 15) im Ausgangszustand vom Außenkörper (8) verdeckt, und insbesondere nicht sichtbar, ist/sind.

9. Vorrichtung nach einem der Ansprüche 1 bis 8, **dadurch gekennzeichnet, dass** die Markierung (16) an einem Ende des Außenkörpers (8), das in Richtung des mit dem Innenkörper (9) verbundenen Endes (6, 7) des Zugelementes (5) ragt, angeordnet ist, insbesondere dass die Markierung (16) der Abschlussrand dieses Endes (6, 7) des Zugelementes (5) ist.

10. Vorrichtung nach einem der Ansprüche 1 bis 9, **dadurch gekennzeichnet, dass** der Anzeigebereich (14) optisch hervorgehoben ist, indem er insbesondere in einer Signalfarbe eingefärbt ist bzw. ggf. dass die Anzeigebereiche (14, 15) optisch hervorgehoben sind, indem sie insbesondere in einer jeweils anderen Signalfarbe eingefärbt sind.

11. Vorrichtung nach einem der Ansprüche 1 bis 10, **dadurch gekennzeichnet, dass** die Manschette (3) einen bandförmigen Gurtbereich (17), mit dem ein Bein (2) oberhalb des Fußes (4) zumindest teilweise umschließbar ist, und einen damit verbundenen bandförmigen Rückenbereich (18), der hinten an der Ferse des Fußes (4) anordenbar ist, aufweist, und dass der Rückenbereich (18) an einem vom Gurtbereich (17) beabstandeten Ende (19) des Rückenbereiches (18) mit dem Zugelement (5) verbunden ist.

12. Vorrichtung nach Anspruch 11, **dadurch gekennzeichnet, dass** der Gurtbereich (17) in seinem Umfang veränderbar ist, sodass ein variabel großer Bereich vom Gurtbereich (17) eng umschließbar ist, insbesondere indem der Gurtbereich (17) durch einen Gurt gebildet ist, der mit Hilfe eines Verschlusses (23), wie einer Schnalle oder einem Klettverschluss, enger oder weiter stellbar ist.

13. Vorrichtung nach Anspruch 11 oder 12, **dadurch gekennzeichnet, dass** der Rückenbereich (18) eine Aussparung (24) mit verringerter Materialstärke aufweist, in die bei einer das Bein (2) zumindest teilweise umschließenden Manschette (3) die Ferse des Fußes (4) platzierbar ist.
